# EUROPEAN PATENT SPECIFICATION

(11) **EP 0 824 901 B1**
(45) Date of publication and mention of the grant of the patent: **01.12.2004**
(21) Application number: 97250238.9
(22) Date of filing: 14.08.1997
(51) Int. Cl.: A61F 2/06, A61B 17/11

(54) **Internal shield for a vascular anastomosis**
Innenschutz für eine vaskuläre Anastomose
Protecteur interne pour un anastomose vasculair

(30) Priority: 21.08.1996 CZ 246196
(43) Date of publication of application: 25.02.1998
(73) Proprietor: MUDR. MILAN KRAJICEK CSc., CS-140 00 Praha 4 (CS)
(72) Inventor: MUDR. MILAN KRAJICEK CSc., CS-140 00 Praha 4 (CS)
(74) Representative: Meissner, Peter E., Dipl.-Ing.

(56) References cited:
- EP-A- 0 709 066
- US-A- 4 313 231
- US-A- 5 156 619

## Description

### Field of the invention

The internal shield of an anastomosis in accordance with the invention solves the problem of the origin of so called intimo-medial hyperplasia, which quite often develops in an anastomosis in a vascular system, particularly in the case of vascular reconstructions of smaller calibre, and leads to the resulting occlusion of the said reconstruction.

### Background of the invention

The anastomosis in the vascular system is basically an artificially created connection either of proper vessels; either of a vessel and a biological or artificial arterial substitute and most commonly is effected by suturing with the use of a special suturing material. Such anastomosis can be created in any part of the vascular system regardless of its calibre. In most cases the connection end to the side is desirable or preferred.

The document EP 0 709 066 A1 describes a synthetic vascular prosthesis which is formed by a first tube member and a second tube member. Both tube members have inner flow paths for blood. An end of the second tube member is connected with an outer surface of the first tube member, and the inner flow path of the second tube member is communicated with the inner flow path of the first tube member.

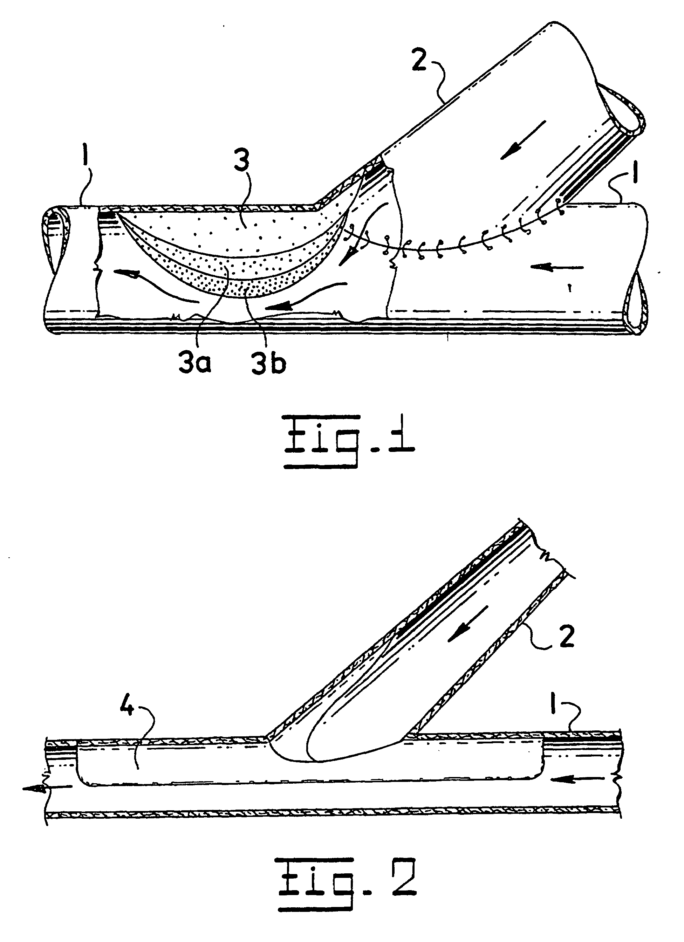

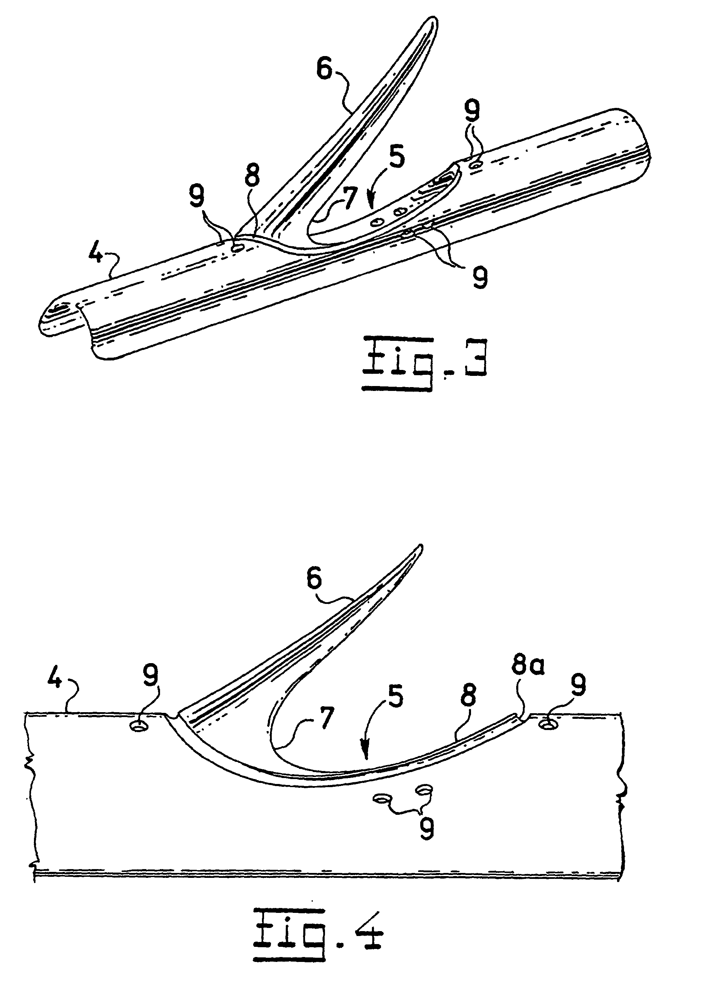

In the case of anastomoses in small calibre area, which generally means a diameter up to 6 mm (for example the reconstruction of coronary arteries), there is a certain problem, occurring relatively often after some time-period, usually after several months, and this problem is not dependent on the technical quality of the anastomosis performed. The said problem is a phenomenon commonly described as the intimo-medial hyperplasia. It means that in the connection, most commonly in the apex of the anastomosis, there gradually develops a mound consisting of structures of the hyperplasic intima and media of the targeted vessel, i.e. the vessel on which the connection is created in the direction of a blood flow. The situation is best illustrated in Fig. 1., which presents a schematic drawing of the anastomosis of the vessel (1) and vascular substitute (2) end to the side with the typical localization of the intimo-medial hyperplasia (3); the blood flow is indicated by the arrow 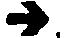 .

This gradually growing structure (3a, 3b) clearly results in the stenosis of the cross-section of the reconstruction, reduction of the blood flow and in its complete occlusion.

The generally accepted theory of the development of the above-described phenomenon is as follows: the created anastomosis disturbs, in dependence on different circumstances, the different characteristics of blond flow, most commonly the so called stability of the flow, which is expressed by Reynolds' number. The anastomosis also disturbs the normal laminar flow towards turbulence. In the direct relation the higher turbulence means also the higher quantity of liberated energy. The forces, which activate the perivascular transfer of the energy by a vibration across the vascular wall, simultaneously cause the chronic stress and damage of the surrounding tissue and at the same time initiate the response of biochemical mediators whose effects finally lead to the gradual development of the intimo-medial hyperplasia.

At the present time no method is known in the accessible literature, which could prevent this undesirable biological process with some degree of reliability. The most common preventive measure is the creation of maximally wide anastomosis. This possibility is of course objectively limited by the calibre of connected tubes, vessels, and vessel and substitute, respectively. The internal reinforcement of the created anastomosis by a stiff lining in accordance with the invention eliminates the principal cause of the intimo-medial hyperplasia - the perivascular transfer of the energy liberated by the turbulence across a soft vascular wall with the permanent vibratory stress and subsequent tissue reaction.

### Description of the invention

The device according to the present invention is presented by the semitubular formation of the diameter commensurate with the diameter of the targeted vessel or substitute, created from a substantially stiff, biologically inert, unmoistenable, non-resorbable, sterilizable single or composite material, having the form of the incomplete two thirds of a tube (4) with a length that is at least five times the diameter of the complete tube and in which between the third and fourth fifth of the length, counter to the blood flow, an orifice (5) is formed on the convexity of the tube, which has a length at least double that of the diameter of the complete tube and which has a width which is at least one quarter of the circumference of the tube, while from the orifice (5) one or two gradually tapered tongue-like structures (6, 10) grow up, wherein each of the tongue-like structures (6, 10) grows up from the orifice (5) in an angle of 20 to 45 degrees counter to the course of the blood flow, has a base with a width that is at least one quarter of the circumference of the tube and gradually transits by its foot (7 or 11) into the margin (8) of the orifice (5), so that the whole transition does not exceed a third of the orifice (5) length and the whole of said tongue-like structure (6, 10) is in the longitudinal axis moderately bow-like curved extemally, as well as the margin (8) of the orifice (5) which is also moderately curved externally and where all edges of the device are blunt and the whole intemal surface of the device is highly polished, and where the tongue-like structures (6 and 10) grow up from the apex of the orifice (5) and from the rear end of the orifice (5), respectively.

In a preferred embodiment of the invention, said tongue-like structure (6) grows up from the apex of the orifice (5) and the rear end of the orifice (5) is free of the tongue-like structure (10).

In some rare cases the development of the intimo-medial hyperplasia occurs on the rear of the anastomosis (counter to the blood flow). Thus, in another preferred embodiment of the invention said tongue-like structure (10) grows up from the rear end of the orifice (5) and the apex of the orifice (5) is free of the tongue-like structure (6).

In yet another preferred embodiment of the invention said tongue-like structure (6) grows up from the apex of the orifice (5) and the tongue-like structure (10) grows up simultaneously from the rear end of the orifice (5).

Furthermore, in the middle of the length of the orifice (5) on both sides, like in the front of the apical end of the orifice (5) and also dorsally from the rear end of the orifice (5) in the appropriate distance, usually in a distance of at least 2 mm, from the top of margin (8) are placed pairs of small holes (9) for the fitting of fixation sutures between the device and the targeted vessel or substitute. These pairs of small holes serve for fixation sutures on the targeted vessel or substitute in such a way that these fixating sutures can be employed in a sufficient distance from the edges of an open vessel, so the anastomosis can be executed distally from the orifice of the device.

In the same way and for the same reason the pairs of small holes can also be placed close to the tips of the tongue-like structures (6) and/or (10).

The present invention further encompasses any of the above-mentioned devices whose external surface is equipped with the physically or chemically bound pharmacologically active agent.

Another aspect of the present invention is the biological or artificial vascular substitute for vascular anastomosis wherein any of the above-mentioned devices is provided as its integral part.

In all cases the material used has to be non-resorbable, sterilizable, biologically inert, unmoistenable, stiff or moderately elastic either by itself (stainless steel, carbonic steel, carbonic composites, glass, or synthetic materials) or in a different physical or chemical combination, most commonly in the form of coating (stainless steel coated by graphite, synthetic base coated by titan etc.).

Simultaneously with the securing of the proper structural stability, the device is maximally thin-walled with all edges blunt.

### Brief description of the drawings

Figure 1 schematically illustrates the typical localization of intimo-medial hyperplasia.
Figure 2 schematically shows the typical locating of the internal shield in an anastomosis.
Figure 3 illustrates the whole device of the invention with its typical characteristics.
Figure 4 illustrates the detail of the margin of the orifice and of the base of the tongue-like structure.

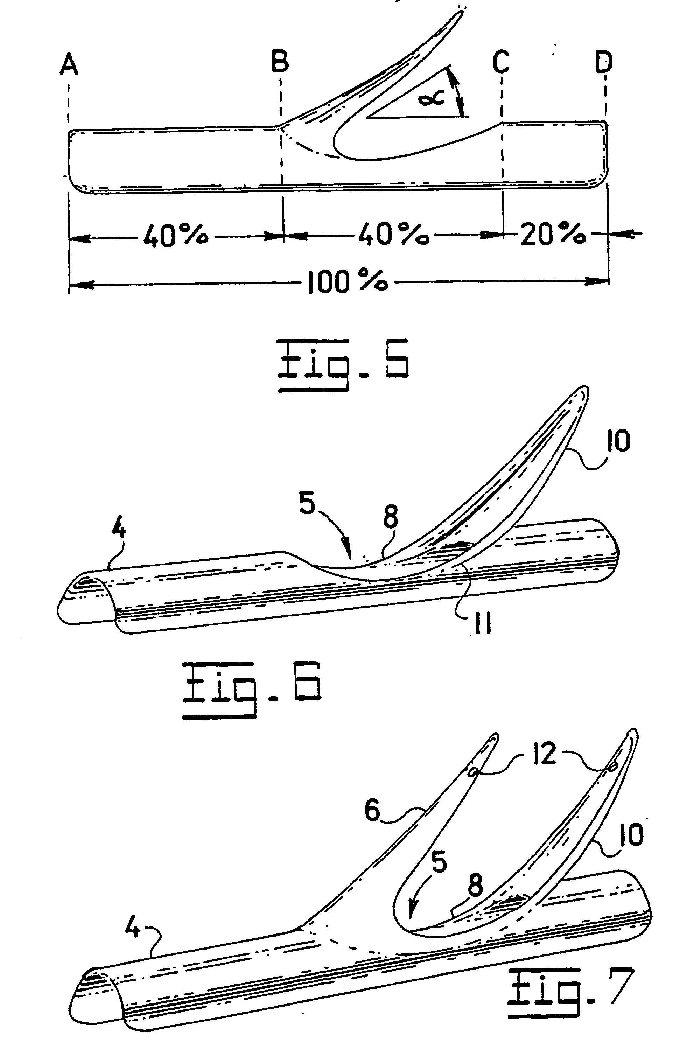

Figure 5 illustrates the dimensional relation of individual parts of the device and the angle of the growing of the tongue-like structure.
Figure 6 illustrates the device with the tongue-like structure growing from the rear end of the orifice.
Figure 7 illustrates the device with the tongue-like structures growing from the apex as well as the rear end of the orifice.

### Examples

1. From the thin-walled carbonic steel is formed an incomplete tube (4) in the form of two thirds of the circumference of diameter 6 mm. This incomplete tube forms the body (4) of the device and its entire length is at least 30 mm, when the first two fifths of the length are entire, as well as the last fifth. In the third and fourth fifth is, on the convexity of the tube, formed the elliptic orifice (5) of width of at least 5 mm as measured on the circumference. From the apical end of this orifice (5) grows up under the angle of 20 - 45 degrees the tongue-like structure (6) at the base wide of at least 5 mm, moderately bow-like curved externally and gradually tapered toward the tip with the minimal length in the central axis of 12 mm. This tongue-like structure (6) is at its base moderately widened and transits gradually by its foot (7) into the margin (8) of the orifice (5). Outside the margin (8) of the orifice (5) are in a distance of at least 2 mm located pairs of small holes (9) for the placing of the fixation sutures on the wall of the targeted vessel or substitute. The whole internal surface is highly polished and the edges of the device are blunt.
2. The device with all characteristics of the device under point 1, but the margins (8) of the orifice (5) are formed in such way that they are moderately turned externally (8a) and by the same way the external surface of the base of the tongue-like structure (6) is adjusted.
3. The device manufactured from the thin-walled stainless steel with the body (4) at least 30 mm long, where from the rear end of the orifice (5) grows up the tongue-like structure (1C) in an angle of 30 degrees counter to the blond flow. The whole internal surface of the device is coated by a thin layer of graphite.
4. The device manufactured from teflon, whose body (4) is at least 30 mm long and from the margin (8) of the orifice (5) grows up one tongue-like structure (6) from the apical end counter to the blond flow under an angle of 30 degrees and of a length of at least 12 mm and from the rear end of the same orifice (5) grows up a similar tongue-like structure (10) under the identical angle and with the same length as the tongue-like structure (6) of the said device. The whole internal surface of the shield is coated by a thin layer of titan and is highly polished.

## Claims

1. A device for the internal shielding of a vascular end to side anastomosis **characterised in that** it is created from a substantially stiff biologically inert, unmoistenable, non-resorbable, sterilizable single or composite material, having the form of the incomplete two thirds of a tube (4) with a length that is at least five times the diameter of the complete tube and in which between the third and fourth fifth of the length, counter to the blood flow, an orifice (5) is formed on the convexity of the tube, which has a length at least double that of the diameter of the complete tube and which has a width which is at least one quarter of the circumference of the tube, while from the orifice (5) one or two gradually tapered tongue-like structures (6, 10) grow up, wherein each of the tongue-like structures (6, 10) grows up from the orifice (5) at an angle of 20 to 45 degrees counter to the course of the blood flow, has a base with a width that is at least one quarter of the circumference of the tube and gradually transits by its foot (7 or 11) into the margin (8) of the orifice (5), so that the whole transition does not exceed a third of the orifice (5) length and the whole of said tongue-like structure (6, 10) is in the longitudinal axis moderately bow-like curved extemally, as well as the margin (8) of the orifice (5) which is also moderately curved extemally and where all edges of the device are blunt and the whole internal surface of the device is highly polished, and where the tongue-like structures (6 and 10) grow up from the apex of the orifice (5) and from the rear end of the orifice (5), respectively.

2. The device according to claim **1, characterised in that** said tongue-like structure (6) grows up from the apex of the orifice (5) and the rear end of the orifice (5) is free of the tongue-like structure (10).

3. The device according to claim **1, characterised in that** said tongue-like structure (10) grows up from the rear end of the orifice (5) and the apex of the orifice (5) is free of the tongue-like structure (6).

4. The device according to claim **1**, **characterised in that** said tongue-like structure (6) grows up from the apex of the orifice (5) and the tongue-like structure (10) grows up simultaneously from the rear end of the orifice (5).

5. The device according to claim **1, 2, 3** or **4**, **characterised in that** in the middle of the length of the orifice (5) on both sides, like in the front of the apical end of the orifice (5) and also dorsally from the rear end of the orifice (5) in the distance at least 2 mm from the top of margin (8) are placed pairs of small holes (9) for the fitting of fixation sutures between the device and the targeted vessel or substitute.

6. The device according to claim **1, 2, 3, 4** or **5 characterised in that** close to the tips of the tongue-like structures (6) and/or (10) are placed pairs of small holes (12) for the fitting of the fixation sutures between these tongue-like structures (6 and/or 10) and the vessel or the substitute into which they are slipped.

7. The device according to claim **1, 2, 3, 4, 5** or **6**, **characterised in that** the internal surface of the said device is equipped with the physically or chemically bound pharmacologically active agent.

8. The device according to daim **1, 2, 3, 4, 5, 6** or **7, characterised in that** the external surface of the device is equipped with the physically or chemically bound± pharmacologically active agent.

9. The biological or artificial vascular substitute for vascular anastomosis **characterised in that** the device according to any of claims **1** to **8** is provided in the said substitute as its integral part by its manufacturer.

## Patentansprüche

1. Vorrichtung für den Innenschutz einer End-zu-Seit-Anastomose eines Gefäßes, **dadurch gekennzeichnet, dass** sie aus einem im Wesentlichen starren biologisch inerten, nicht befeuchtbaren, nicht resorbierbaren, sterilisierbaren Einstoff- oder Verbundmaterial gefertigt ist, das die Form von unvollständigen zwei Drittel einer Röhre (4) mit einer Länge aufweist, die mindestens das Fünffache des Durchmessers der vollständigen Röhre beträgt und in der zwischen dem dritten und dem vierten Fünftel der Länge entgegen dem Blutstrom auf der konvexen Seite der Röhre eine Einmündung ausgebildet ist, deren Länge mindestens das Doppelte des Durchmessers der vollständigen Röhre und deren Breite mindestens ein Viertel des Röhrenumfangs beträgt, während aus der Einmündung (5) ein oder zwei sich allmählich verjüngende zungenförmige Gebilde (6, 10) hervorgehen, worin jedes der zungenförmigen Gebilde (6,10) aus der Einmündung (5) in einem Winkel von 20 bis 45 Grad entgegen der Richtung des Blutstroms hervorgeht, eine Grundfläche mit einer Breite von mindestens einem Viertel des Röhrenumfangs hat und allmählich mit ihrem unteren Ende (7 oder 11) in den Rand (8) der Einmündung (5) übergeht, so dass der gesamte Übergang ein Drittel der Länge der Einmündung nicht überschreitet und das gesamte zungenförmige Gebilde (6, 10) in der Längsachse eine mäßig bogenförmige Außenkrümmung aufweist ebenso wie der Rand (8) der Einmündung (5), der ebenfalls eine mäßige Außenkrümmung aufweist, und alle Ränder der Vorrichtung abgestumpft sind und die gesamte Innenoberfläche der Vorrichtung stark poliert ist und wo die zungenförmigen Gebilde (6 und 10) aus dem Scheitelpunkt der Einmündung (5) bzw. dem Ende der Einmündung (5) hervorgehen.

2. Vorrichtung nach Anspruch 1, **dadurch gekennzeichnet, dass** das zungenförmige Gebilde (6) aus dem Scheitelpunkt der Einmündung (5) hervorgeht und das hintere Ende der Einmündung (5) frei von dem zungenförmigen Gebilde (10) ist.

3. Vorrichtung nach Anspruch 1, **dadurch gekennzeichnet, dass** das zungenförmige Gebilde (10) aus dem hinteren Ende der Einmündung hervorgeht und der Scheitelpunkt der Einmündung (5) frei von dem zungenförmigen Gebilde (6) ist.

4. Vorrichtung nach Anspruch 1, **dadurch gekennzeichnet, dass** das zungenförmige Gebilde (6) aus dem Scheitelpunkt der Einmündung (5) hervorgeht und das zungenförmige Gebilde (10) gleichzeitig aus dem hinteren Ende der Einmündung (5) hervorgeht.

5. Vorrichtung nach Anspruch 1, 2, 3 oder 4, **dadurch gekennzeichnet, dass** in der Mitte in der Länge der Einmündung (5) auf beiden Seiten, sowohl vor dem Scheitelpunkt der Einmündung (5) wie auch dorsal vom hinteren Ende der Einmündung (5), paarweise kleine Löcher (9) zum Einbringen von Befestigungsnähten zwischen der Vorrichtung und dem Zielgefäß bzw. dem Gefäßersatz angeordnet sind.

6. Vorrichtung nach Anspruch 1, 2, 3, 4 oder 5, **dadurch gekennzeichnet, dass** in der Nähe der Spitzen der zungenförmigen Gebilde (6) und/oder (10) paarweise kleine Löcher (12) zum Einbringen von Befestigungsnähten zwischen diesen zungenförmigen Gebilden (6 und/oder 10) und dem Gefäß bzw. dem Gefäßersatz, in das/den es eingeschoben wird, angeordnet sind.

7. Vorrichtung nach Anspruch 1, 2, 3, 4, 5 oder 6, **dadurch gekennzeichnet, dass** die innere Oberfläche der Vorrichtung mit dem physikalisch oder chemisch gebundenen pharmakologisch wirksamen Mittel versehen ist.

8. Vorrichtung nach Anspruch 1, 2, 3, 4, 5, 6 oder 7, **dadurch gekennzeichnet, dass** die äußere Oberfläche der Vorrichtung mit dem physikalisch oder chemisch gebundenen pharmakologisch wirksamen Mittel versehen ist.

9. Biologischer oder künstlicher Gefäßersatz für die Gefäßanastomose, **dadurch gekennzeichnet, dass** die Vorrichtung nach jedem der Ansprüche 1 bis 8 vom Hersteller als wesentlicher Bestandteil des Gefäßersatzes zur Verfügung gestellt wird.

## Revendications

1. Dispositif pour la protection interne d'une anastomose vasculaire termino-latérale, **caractérisé en ce qu'**il est fabriqué à partir d'un matériau simple ou composite sensiblement rigide, biologiquement inerte, non-humectable, non-résorbable, stérilisable, ayant la forme des deux tiers incomplets d'un tube (4) avec une longueur qui est au moins égale à cinq fois le diamètre du tube complet et dans lequel entre le troisième et le quatrième cinquième de la longueur, en opposition au flux sanguin, un orifice (5) est formé sur la convexité du tube, qui a une longueur au moins égale au double de celle du diamètre du tube complet et qui a une largeur qui est au moins égale à un quart de la circonférence du tube, tandis qu'à partir de l'orifice (5) une ou deux structures comme des languettes progressivement tronconiques (6, 10) s'élèvent, dans lequel chacune des structures comme des languettes (6, 10) s'élèvent à partir de l'orifice (5) à un angle de 20 à 45 degrés en opposition à la circulation du flux sanguin, a une base avec une largeur qui est au moins égale à un quart de la circonférence du tube et progressivement transite par son pied (7 ou 11) dans le bord (8) de l'orifice (5), de sorte que la transition complète n'excède pas un tiers de la longueur de l'orifice (5) et que la totalité de ladite structure comme une languette (6, 10) est dans l'axe longitudinal modérément comme un arc courbée vers l'extérieur, tout comme le bord (8) de l'orifice (5) qui est aussi modérément courbé vers l'extérieur et dans lequel les rebords du dispositif sont peu tranchants et la totalité de la surface interne du dispositif est parfaitement polie, et dans lequel les structures comme des languettes (6 et 10) s'élèvent à partir de l'apex de l'orifice (5) et à partir de l'extrémité arrière de l'orifice (5), respectivement.

2. Dispositif selon la revendication 1, **caractérisé en ce que** ladite structure comme une languette (6) s'élève à partir de l'apex de l'orifice (5) et l'extrémité arrière de l'orifice (5) ne comprend pas la structure comme une languette (10).

3. Dispositif selon la revendication 1, **caractérisé en ce que** ladite structure comme une languette (10) s'élève à partir de l'extrémité arrière de l'orifice (5) et l'apex de l'orifice (5) ne comprend pas la structure comme une languette (6).

4. Dispositif selon la revendication 1, **caractérisé en ce que** ladite structure comme une languette (6) s'élève à partir de l'apex de l'orifice (5) et la structure comme une languette (10) s'élève simultanément à partir de l'extrémité arrière de l'orifice (5).

5. Dispositif selon la revendication 1, 2, 3 ou 4, **caractérisé en ce que** dans le milieu de la longueur de l'orifice (5) sur les deux côtés, comme à l'avant de l'extrémité apicale de l'orifice (5) et aussi dorsalement à partir de l'extrémité arrière de l'orifice (5) à une distance d'au moins 2 mm à partir du haut du bord (8) sont placées des paires de petits trous (9) pour accueillir des sutures de fixation entre le dispositif et le vaisseau ou le substitut visé.

6. Dispositif selon la revendication 1, 2, 3, 4 ou 5, **caractérisé en ce que** à proximité des bouts des structures comme des languettes (6) et/ou (10) sont placées des paires de petits trous (12) pour accueillir les sutures de fixation entre ces structures comme dès languettes (6 et/ou 10) et le vaisseau ou le substitut dans lequel elles sont glissées.

7. Dispositif selon la revendication 1, 2, 3, 4, 5 ou 6, **caractérisé en ce que** la surface interne dudit dispositif est équipée d'agent pharmacologiquement actif physiquement ou chimiquement lié.

8. Dispositif selon la revendication 1, 2, 3, 4, 5, 6 ou 7, **caractérisé en ce que** la surface externe du dispositif est équipé de l'agent pharmacologiquement actif lié physiquement ou chimiquement.

9. Substitut vasculaire biologique ou artificiel pour une anastomose vasculaire, **caractérisé en ce que** le dispositif selon l'une quelconque des revendications 1 à 8 est proposé dans ledit substitut en tant que partie intégrante par son fabricant.
